(19) Europäisches Patentamt
European Patent Office
Office européen des brevets

(11) **EP 2 708 186 A1**

(12) **EUROPÄISCHE PATENTANMELDUNG**

(43) Veröffentlichungstag:
**19.03.2014  Patentblatt 2014/12**

(51) Int Cl.:
***A61B 5/22*** *(2006.01)*      *A63B 24/00* *(2006.01)*

(21) Anmeldenummer: **13184213.0**

(22) Anmeldetag: **12.09.2013**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**

(30) Priorität: **12.09.2012   AT 503802012**

(71) Anmelder: **Schimpl, Bernhard
4040 Lichtenberg (AT)**

(72) Erfinder: **Schimpl, Bernhard
4040 Lichtenberg (AT)**

(74) Vertreter: **Jell, Friedrich
Bismarckstrasse 9
4020 Linz (AT)**

(54) **Verfahren zur Festlegung und/oder Steuerung des körperlichen Trainings eines Probanden**

(57)     Es wird ein Verfahren zur Festlegung und/oder Steuerung des körperlichen Trainings eines Probanden auf Grundlage wenigstens einer Untersuchung seiner körperlichen Leistungsfähigkeit gezeigt, bei welcher Untersuchung Beschleunigungen (a) bei unterschiedlichen Trainingswiderständen, insbesondere Beschleunigungen (a) unterschiedlicher Massen (m), erfasst werden und das Training in Abhängigkeit einer ermittelten Extremstelle (2) aus einer mathematischen Funktion (F), abhängig von diesen Trainingswiderständen, insbesondere der Größe der jeweiligen Masse (m), und den dazu erfassten Beschleunigungen (a), festgelegt und/oder gesteuert wird. Zur Optimierung des Trainingseffekts bzw. um eventuelle Über- oder Fehlbelastungen des Körpers zu vermeiden, wird vorgeschlagen, dass in der Funktion (F) von den erfassten Beschleunigungswerten ausschließlich ein von diesen abhängiger Maximalwert ($a_{max}$, $á_{max}$) herangezogen wird.

*FIG.1*

EP 2 708 186 A1

**Beschreibung**

Die Erfindung betrifft ein Verfahren zur Festlegung und/oder Steuerung des körperlichen Trainings eines Probanden auf Grundlage wenigstens einer Untersuchung seiner körperlichen Leistungsfähigkeit, bei welcher Untersuchung Beschleunigungen bei unterschiedlichen Trainingswiderständen, insbesondere Beschleunigungen unterschiedlicher Massen, erfasst werden und das Training in Abhängigkeit einer ermittelten Extremstelle aus einer mathematischen Funktion, abhängig von diesen Trainingswiderständen, insbesondere der Größe der jeweiligen Masse, und den dazu erfassten Beschleunigungen, festgelegt und/oder gesteuert wird.

Aus dem Stand der Technik sind Verfahren zur Festlegung und/oder Steuerung des körperlichen Trainings eines Probanden bekannt, zu deren Zweck Untersuchungen anhand von Beschleunigungen unterschiedlicher Massen durchgeführt werden. So zeigt die US 2011/0207581 A1 ein Verfahren, bei welchem die Leistung eines Probanden - unter anderem mithilfe eines Accelerometers - bestimmt und als mathematische Funktion dargestellt wird. Eine Extremstelle dieser Funktion aus kennzeichnet die maximale Leistung des Probanden - ohne differenzierter Berücksichtigung der Beschleunigungswerte. Anhand dieser Extremstelle wird versucht, auf Parameter für völlig unterschiedliche Trainingsformen (Schnellkrafttraining, Muskelhypertrophietraining etc.) hinsichtlich der Größe der zu beschleunigenden Masse, der Geschwindigkeit der durchzuführenden Übung und der Anzahl an Wiederholungen rückzuschließen.

Die US2009/0069722A1 erwähnt, dass während der Durchführung einer Übung unterschiedlichen Beschleunigungswerte erreicht werden - auch eine maximale Momentanleistung, was jedoch als fehleranfällig betrachtet wird.

Die US2012/0094804A1 handelt von einer Anzeigevorrichtung, die unter anderem ein Produkt aus maximaler Beschleunigung $a_{max}$ und Masse anzeigt. Ein Verfahren zur Festlegung und/oder Steuerung des körperlichen Trainings eines Probanden fehlt der US2012/0094804A1. Daran ändert auch die WO2007/036611A1 nicht, die offenbart, mithilfe einer Bestimmung der maximalen Beschleunigung einer Masse eines Probanden auf die Masse, die dieser maximal hochheben könnte, rückzuschließen.

Die Erfindung hat sich daher die Aufgabe gestellt, ein Verfahren ausgehend vom Stand der Technik derart zu verbessern, dass dieses eine genaue Untersuchung der körperlichen Leistungsfähigkeit erlauben und das Training in seiner Effektivität - insbesondere auch hinsichtlich unterschiedlicher Trainingsziele - optimieren kann. Es soll vor allem auch Berücksichtigung finden, ein Training unter den hierfür idealen Vorgaben durchzuführen, um etwa ineffektives Training, Übertraining, Fehlbelastungen und deren negative Auswirkungen zu verhindern.

Die Erfindung löst die gestellte Aufgabe dadurch, dass in der Funktion von den erfassten Beschleunigungswerten ausschließlich ein von diesen abhängiger Maximalwert herangezogen wird.

Wird in der Funktion von den erfassten Beschleunigungswerten ausschließlich ein von diesen abhängiger Maximalwert herangezogen, kann unter anderem eine wesentlich exaktere Untersuchung der körperlichen Leistungsfähigkeit eines Probanden erfolgen. Erfindungsgemäß können nämlich Faktoren wie etwa eine nicht korrekte Ausführung einer Übung, also einer Beschleunigung einer Masse, in einer Untersuchung wesentlich besser berücksichtigt werden. Diesem Vorteil kann auch insofern entscheidende Bedeutung zukommen, weil beispielsweise die Leistungsfähigkeit des Bewegungsapparats der linken im Vergleich zur rechten Körperhälfte sehr häufig unterschiedlich gut ausgeprägt ist, was bis zu einem gewissen Grad, etwa durch Abweichungen vom vorgegebenen/vorgesehenen Bewegungsablauf oder dadurch, dass Muskel einer Körperseite mehr Leistung erbringen, kompensiert werden kann. Verfahren entsprechend dem Stand der Technik können diese Faktoren nur unzureichend erkennen und berücksichtigen, da in diesen lediglich Parameter, wie Geschwindigkeit der Übungsausführung etc., berücksichtigt werden. Derartige Verfahren können also dazu führen, dass eine Körperseite bevorzugt trainiert, schwächere Muskelgruppen überlastet werden etc., wodurch die willkürliche neuromuskuläre Aktivierungsfähigkeit, also das Zusammenspiel zwischen Nerven und Muskulatur beeinträchtigt und sogar verschlechtert werden kann. Dadurch, dass im erfindungsgemäßen Verfahren in der Funktion von den erfassten Beschleunigungswerten jedoch ausschließlich ein von diesen abhängiger Maximalwert - zum Beispiel die maximale Beschleunigung oder der maximale Wert der nach der Zeit abgeleiteten Beschleunigungen - herangezogen wird und Voraussetzung für hohe maximale Werte ist, dass alle an der Ausführung einer Trainingsübung beteiligten Muskeln, Nervenbahnen etc. vorteilhaft zusammenwirken, können also genaue Rückschlüsse auf die körperliche Leistungsfähigkeit erfolgen. Es kann somit ermöglicht werden, ein ausgewogenes Training mit maximaler Effektivität festzulegen und/oder zu steuern, wobei negative Trainingseffekte wie etwa Überbelastungen schwächerer Muskeln und daraus möglicherweise folgende Verletzungen - etwa aufgrund dadurch verursachter Dehnungsbelastung oder Fehlbelastung des Bewegungsapparats - reduziert werden. Dadurch, dass also beispielsweise unterschiedlich leistungsfähige oder mit unterschiedlichen Zielen trainierte Muskel eines Probanden berücksichtigt werden, kann unter anderem eine Annäherung bzw. ein Ausgleich der Leistungsfähigkeiten von Muskel beider Körperseiten bzw. bei einer Übung synergistisch zusammenwirkender Muskeln erreicht werden. Erfindungsgemäß kann also dafür Sorge getragen werden, dass der Informationsgehalt einer Überprüfung der körperlichen Leistungsfähigkeit und in weiterer Folge die Qualität eines Trainings optimiert werden, womit dieses selbst für jene Personen anwendbar ist, deren körperlichen Voraussetzungen eingeschränkt sind - also etwa für Personen, die ein Rehabilitationstraining durchführen.

Des Weiteren können vorteilhaft auch jene Parameter für ein Training bestimmt werden, durch die eine besonders gute Verbesserung der Leistungsfähigkeit erreicht werden kann - und zwar insbesondere spezifisch hinsichtlich Kraft-Ausdauer, in weiterer Folge aber auch für Schnellkraft. Es hat sich nämlich herausgestellt, dass der gemeinsamen Berücksichtigung der richtigen Wahl der Größe des zu überwindenden Widerstands, insbesondere einer zu beschleunigenden Masse, sowie der Durchführung einer Übung mit dem Ziel, stets von Beschleunigungswerten abhängige Maximalwerte, zum Beispiel maximale Beschleunigung oder der maximale Wert der nach der Zeit abgeleiteten Beschleunigungen, zu erreichen, entscheidende Bedeutung zukommt. Dabei spielt auch nur eine untergeordnete Rolle, mit welchen Erfordernissen - etwa hinsichtlich der Größe einer zu beschleunigenden Masse - letztendlich eine Sportart oder ein Wettkampf tatsächlich ausgeführt werden soll. Ebenso wurde festgestellt, dass ab einem bestimmten Ermüdungsgrad die von Beschleunigungswerten abhängige Maximalwerte gegen einen Widerstand abrupt abfällt. Es dürften ab einem bestimmten Trainingszeitpunkt also vermehrt andere Muskelfasertypen und/oder Teile des innervierenden Nervensystems in die Erbringung eines bestimmten Trainingsleistung miteinbezogen werden, die andere Eigenschaften aufweisen - etwa bevorzugt zur Erbringung von Maximalkraft. Der Identifizierung dieses Trainingszeitpunkts kommt entscheidende Bedeutung zu, da ab diesem nicht nur der Trainingseffekt sinkt, vielmehr kann ein über diesen Schwellenwert hinaus weitergeführtes Training in unerwünschte Belastungsintensitäten abgleiten und sich sogar negativ auf den Trainingserfolg auswirken, ebenso kann eine übermäßige Erschöpfung und eine dadurch erforderliche verlängerte Regenerationsphase vermieden werden - erfindungsgemäß wird das Training daher unter Berücksichtigung dieses Schwellwerts ab- oder unterbrochen. Diese Faktoren stellen eine wesentliche Verbesserung gegenüber Verfahren des Stands der Technik dar, in denen lediglich die Gesamtleistung, die Geschwindigkeit einer Übungsausführung etc. herangezogen werden und versucht wird, ohne Berücksichtigung der einzelnen Leistungsfaktoren oder deren Verhältnis zueinander, Rückschlüsse auf Trainings mit unterschiedlichen Zielen zu erhalten. Erfindungsgemäß kann also durch Bestimmung der Trainingsparameter, sowie durch eine gezielte Trainingssteuerung eine Erhöhung der Qualität des Trainings zur Verbesserung der neuromuskulären Aktivierbarkeit entsprechend dem gewünschten Trainingsziel erfolgen. Der Vollständigkeit wegen wird festgehalten, dass bei dem erfindungsgemäßen Verfahren der Trainingswiderstand auf verschiedenste Weise vorgegeben werden kann. Hierzu zählt insbesondere eine bestimmte Masse, etwa als Hantel, die gegen die Schwerkraft zu bewegen ist. Ebenso vorstellbar und im Sinne der Erfindung sind Beschleunigungen über Seilzüge, Beschleunigungen gegen die Wirkung von diversen Bremsvorrichtungen, Federn oder Dehnungswiderstände, etwa durch Gummibänder. Des Weiteren sind auch Beschleunigungen des Körpers oder dessen Teile - mit/ohne Zusatzgewicht oder zusätzlichem Widerstand möglich und eingeschlossen. Dementsprechend kann ein Accelerometer an einem Trainingsgegenstand oder auch am Körper eines Probanden befestigt werden.

Wird als Maximalwert die maximale Beschleunigung verwendet, ist jene zu beschleunigende Masse bestimmbar, bei welcher der Körper in Hinblick auf die maximale Beschleunigung zu besonders hoher Kraftentwicklung fähig ist. Dies kann sich unter anderem bei Kraft-Ausdauer-Training als vorteilhaft herausstellen. Aber auch im Zusammenhang mit einer korrekten Ausführung eines Trainings im Kraft-Ausdauer-Bereich ermöglicht wird. Durch die ausschließliche Berücksichtigung der maximalen Beschleunigung kann ein Kraft-Ausdauer-Training also wesentlich gezielter auf die individuelle Leistungsfähigkeit bzw. auf die individuellen Bedürfnisse eines Probanden eingegangen werden. Außerdem kann ein derartiges Verfahren vergleichsweise schnell und einfach durchgeführt werden. Überraschend hat sich vielfach gezeigt, dass die für ein Training festgelegten Massen im Vergleich zum Stand der Technik sich deutlich unterscheiden. Auch kann so erfindungsgemäß eine Ermüdung, die auf ein uneffektives oder sich negativ auswirkendes Training hinweisen würde, wesentlich leichter erkennbar werden.

Mit einer Weiterbildung des Verfahrens, in welchem als Maximalwert der maximale Wert der nach der Zeit abgeleiteten Beschleunigungen verwendet wird, kann sich das Verfahren insbesondere für eine Untersuchung der Leistungsfähigkeit bzw. für eine Festlegung und/oder Steuerung in Hinblick auf ein Schnellkrafttraining eignen. Es findet nämlich gezielt der mit erreichte maximale Ruck Eingang in jene mathematische Funktion, durch die die Trainingsparameter - also der optimale Trainingswiderstand, zu von den Beschleunigen abhängige Maximalwerte, die es zu erreichen gilt etc. - für ein Schnellkrafttraining bestimmt werden. Somit kann die Dynamik, mit der eine Trainingsübung von einem Probanden ausgeführt wird, untersucht und das Training auf die entsprechende neuromuskuläre Aktivierungsfähigkeit abgestimmt werden. Wiederum gilt, dass die Trainingsübungen mit dem Ziel durchzuführen sind, eine den angesprochenen Maximalwert zu erreichen und. Falls diesbezüglich ein Schwellenwert unterschritten wird, ist das Training ab- oder zu unterbrechen. Somit kann ein Training erfolgen, bei dem die Trainingsvorgaben, gezielt auf die individuelle Leistungsfähigkeit eines Probanden abgestimmt sind, um dessen Schnellkraft zu verbessern und negative Trainingseffekte zu vermeiden.

Werden für die Bestimmung des Maximalwerts Abweichungen der Bewegungsbahn am bzw. des Probanden von der Wirkrichtung des zu überwindenden Trainingswiderstands berücksichtigt, kann eine Vielzahl von Trainingsübungen verbessert analysiert und gesteuert werden. Dies kann beispielsweise bei Übungen, bei denen die Beschleunigung einer Masse nicht nur entlang oder annähernd entlang der Falllinie erfolgt, Rotationen beinhaltet usw. Bedeutung haben. So kann etwa ermöglicht werden, ermittelte Beschleunigungswerte in Abhängigkeit zu eventuell im Laufe der Bewegungsbahn einer Übung sinkenden Widerständen zu beurteilen. Vorstellbar wäre zu diesem Zweck die Berücksichtigung eines Korrekturfaktors in der Funktion. Eine andere denkbare Variante ist, lediglich Teile der Bewegungsbahn einer Trainingsübung für das erfindungsgemäße Verfahren zu berücksichtigen - insbesondere jene Teile, die der Wirkrichtung des Trainingswiderstands im Wesentlichen entgegengesetzt sind.

Wird das Training unter Vorgabe eines zu erreichenden Maximalwerts oder eines zu erreichenden Bereichs um diesen festgelegt, kann eine gezielte Steuerung und Kontrolle des Trainings durchgeführt werden. In diesem Zusammenhang hat sich nämlich überraschend gezeigt, dass die Maximalwerte - etwa die maximal erreichte Beschleunigung - mit Eintreten einer bestimmten körperlichen Ermüdung eines Probanden rasch und stark absinkt. Diese Erkenntnis ist insofern von hoher Bedeutung, da das Training nach Überschreiten eines derartigen Ermüdungszustands nicht mehr den Zielsetzungen hinsichtlich des erwünschten Trainingseffekts entspricht. Erfindungsgemäß kann daher auf die körperliche Verfassung eines Probanden während des Trainings wesentlich gezielter reagiert und die Trainingssteuerung optimiert werden. Ebenso wichtig ist natürlich die Möglichkeit, auf diese Weise zu prüfen, ob die Trainingsvorgaben durch den Probanden eingehalten werden - was im Stand der Technik, in dem lediglich die Leistung, die Geschwindigkeit der Übungsausführung etc. berücksichtigt werden, nur eingeschränkt möglich ist. Es hat sich nämlich gerade bei Kraft-Ausdauer- und Schnellkraft-Training gezeigt, dass primär mit jenen Wiederholungen einer Beschleunigung gegen einen Widerstand Fortschritte in der Leistungsfähigkeit erreicht werden können, die mit zumindest annähernd maximalen von der Beschleunigung abhängigen Werten durchgeführt werden. Ein Fortsetzen eines Trainings über die Ermüdungsschwelle hinaus - also bei deutlichem Abfall dieser Werte - kann rasch in Übertraining mit erhöhter Verletzungsanfälligkeit, verlängerter Regenerationszeit etc. münden. Erfindungsgemäß wird ein Training daher unter- oder abgebrochen. Somit ist eine Optimierung der Trainingsqualität durch Bestimmung und Festlegung jener Trainingsbedingungen und -grenzen, innerhalb der vor allem für Schnellkraft- oder Kraftausdauertraining die besten Erfolge erzielbar sind, möglich - unter verbesserter Vermeidung nicht dem Trainingsziel entsprechender Belastungen.

Wird das Training unter Berücksichtigung einer Zeitdauer oder eines Zeitbereichs bis zur Erreichung eines Maximalwerts festgelegt, können die Vorgaben hinsichtlich der Trainingsbelastungen verbessert gesteuert und angepasst werden. Da es bei zahlreichen Sportarten erforderlich ist, etwa eine maximale Beschleunigung bzw. eine maximale Beschleunigung möglichst hoher Widerstände in einer möglichst kurzen Zeitspanne zu erreichen, ist es sinnvoll, ein Schnellkrafttraining, aber auch ein Kraft-Ausdauer-Training dahingehend regelmäßig zu evaluieren. Erfindungsgemäß kann so beispielsweise die Fähigkeit des neuromuskulären Systems, im Zuge der Ausführung einer Trainingsübung Leistung/Arbeit mit hohen bzw. maximalen Beschleunigungswerten möglichst rasch zu erreichen, berücksichtigt werden.

Werden zur Festlegung und/oder Steuerung des körperlichen Trainings eines Probanden Vergleichsdaten anderer Probanden herangezogen, können vorteilhaft Rückschlüsse auf aktuelle Leistungsfähigkeit, Lebensumstände bzw. durch falsche Trainingsbelastungen verursachte negative Auswirkungen etc., evaluiert werden. Dementsprechend ist es selbstverständlich vorstellbar, derartige Analysen verschiedener Probanden oder Probandengruppen nach bestimmten Kriterien zur Entwicklung von Modellen heranzuziehen, um rasch Rückschlüsse auf die Leistungsfähigkeit und Trainingserfordernisse eines Probanden zu erhalten.

In den Zeichnungen wird die Erfindung beispielsweise anhand von Figuren näher erläutert. Es zeigen

Fig. 1      eine grafische Darstellung der Ergebenisse der erfindungsgemäßen Funktionnen bei steigenden Massen,

Fig. 2      eine graphische Darstellung der Ergebenisse einer Funktion von Serien einer Beschleunigung im Zuge eines Trainings un

Fig. 3      eine graphische Darstellung der Zeit bis zur Erreichnug der maximalen Beschleunigung.

Zur Untersuchung der körperlichen Leistungsfähigkeit eines Probanden wird eine Trainingsübung, in diesem Fall Bankdrücken, mit unterschiedlichen Massen nacheinander durchgeführt und die mit der jeweiligen Masse im Zuge der Übungsausführung erreichten Beschleunigungen mittels Acceleromter bestimmt. Die Ergebnisse dieser Untersuchung werden in den Figuren dargestellt. Die Trainingsbelastung wird in diesem Beispiel demnach primär durch eine gegen die Erdanziehung zu beschleunigende Masse festgelegt. Da die Beschleunigung einer Masse beim Bankdrücken praktisch ausschließlich entlang der Falllinie erfolgt, kann ein Korrekturfaktor zur Berücksichtigung einer von dieser abweichenden Bewegungsbahn vernachlässigt werden.

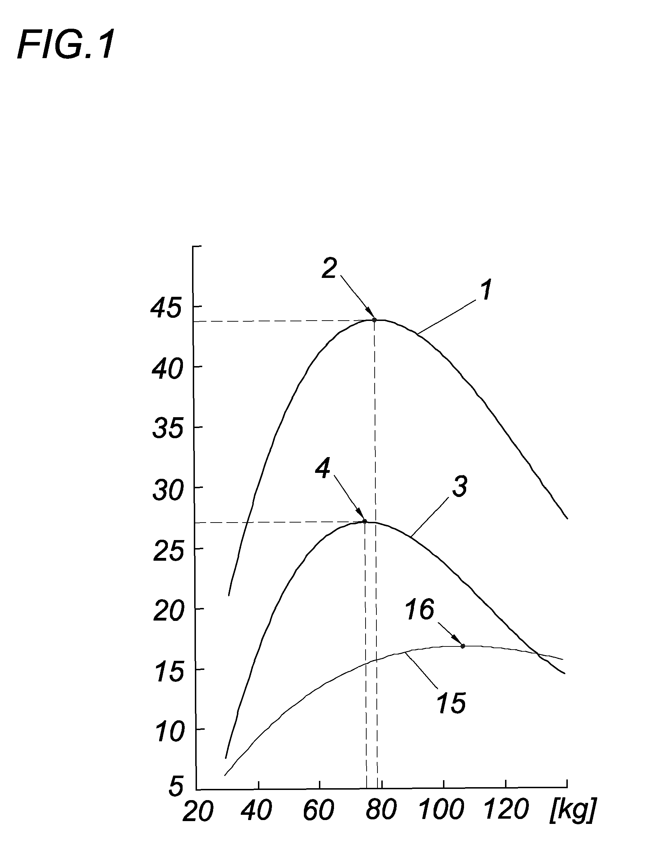

Figur 1 zeigt ein Liniendiagramm, dessen x-Achse die Größe einer zu beschleunigenden Masse und die y-Achse das Ergebnis einer Funktion anzeigt.

Die Kurve 1 zeigt eine Interpolation der Ergebnisse der Funktion

$$F = m * a_{max}$$

wobei F die Funktion,
m die beschleunigte Masse und
$a_{max}$ die im Zuge der Durchführung der Übung maximal erreichte Beschleunigung darstellen.

Diese Kurve 1 lässt erfindungsgemäß eine verbesserte Untersuchung der körperlichen Leistungsfähigkeit eines Probanden zu - und zwar insbesondere hinsichtlich jener im Kraft-Ausdauer-Bereich. Ein Ergebnis der Funktion F, also ein Punkt der Kurve 1, zeigt somit jenen Anteil an der gesamten im Zuge der Beschleunigung a einer Masse m aufgewendeten Kraft, die mit maximaler Beschleunigung $a_{max}$ erreicht werden konnte. Am Beginn der Kurve 1 ist zu erkennen, dass mit geringen Massen kleinere Ergebnisse der Funktion zu erreichen sind, da mit diesen - verglichen mit höheren - Massen nicht überproportional große maximale Beschleunigungswerte erreichbar sind. Somit zeigt die Kurve 1 der Funktion F bei einer zu beschleunigenden Masse von etwa 79 Kilogramm eine Extremstelle 2. Wenig überraschend sinken die Ergebnisse der Funktion F mit steigender Masse nach dieser Extremstelle 2, da deren maximal erreichte Beschleunigung $a_{max}$ überproportional stark abfällt.

Das Training wird also in Abhängigkeit dieser ermittelten Extremstelle 2 aus der mathematischen Funktion F, abhängig von diesen Massen m und deren maximalen Beschleunigungen $a_{max}$, festgelegt und/oder gesteuert. Mithilfe der Extremstelle 2 ist nämlich jene zu beschleunigende Masse bestimmbar, bei welcher der Körper in Hinblick auf die maximale Beschleunigung $a_{max}$ zu besonders hoher Kraftentwicklung fähig ist - diesbezüglich somit auch besonders gefordert ist, womit bei korrekter Ausführung der Übung ein optimales Kraft-Ausdauer-Training ermöglicht wird. Durch die ausschließliche Berücksichtigung der maximalen Beschleunigung $a_{max}$ kann ein Kraft-Ausdauer-Training also wesentlich gezielter auf die individuelle Leistungsfähigkeit bzw. auf die individuellen Bedürfnisse eines Probanden abgestimmt werden. Beispielsweise stellen sich bei Untrainierten oder auch bei Personen, die eine Masse m in einem für sie neuen/ungewohnten Bewegungsablauf beschleunigen sollen, die Extremstelle 2, also das Maximum der Funktion F, bereits bei erheblich niedrigeren Massen ein. In diesen Fällen kann vielfach auch festgestellt werden, dass die Kurve entsprechend Funktion F in ihrem Verlauf auch erhebliche Abweichungen zu jenem der Kurve 1 aufweist. Oft ist die Steilheit ihres Anstiegs bis zur Extremstelle 2 weniger stark, vielfach ist diese auch weniger deutlich ausgeprägt. Hierzu können mithilfe von Vergleichswerten anderer Probanden zusätzliche, aufschlussreiche Hinweise auf die Leistungsfähigkeit erhalten werden.

Kurve 3 der Figur 1 zeigt das interpolierte Ergebnis einer Weiterbildung der Funktion F, in der die Beschleunigungen (a) nach der Zeit abgeleitet werden. Die bei dieser Ableitung erhaltenen Maximalwerte $á_{max}$ fließen in die Funktion F ein:

$$F = m * á_{max}$$

Dadurch, dass auf diese Weise der mit einer Masse m maximale erreichte Ruck Eingang in die mathematische Funktion erhält und gezielt die Dynamik einer Übungsausführung betrachtet wird, kann eine Untersuchung der Leistungsfähigkeit mithilfe dieser weitergebildeten Funktion F insbesondere in Hinblick auf ein Schnellkrafttraining erfolgen. Analog zu obenstehenden Ausführungen können in weiterer Folge gezielt entsprechende Rückschlüsse auf Leistungsfähigkeit bzw. Festlegung und/oder Steuerung des Trainings erfolgen, um einen optimalen Trainingseffekt zu erreichen.

Auch hier kann ein wesentlicher Vorteil gegenüber dem Stand der Technik erreicht werden, da bei diesem lediglich versucht wird, anhand einer ermittelten Gesamtleistung auf unterschiedliche Trainingsformen und erforderlichen Belastungen rückzuschließen. Kurve 15 der Figur 1 zeigt die Auswertung der Ergebnisse in herkömmlieher Weise. Dies wird anhand der Kurve 15 der Figur 1, als interpolierte Ergebnisse aus der Berechnung der Kraft, gebildet aus der beschleunigten Masse und der Gesamtbeschleunigung bzw. des Integrals der Beschleunigungen gezeigt. Es ist erkennbar, dass aussagekräftige Rückschlüsse auf die körperliche Leistungsfähigkeit sowie auf ein optimales Kraft-Ausdauer- oder Schnellkrafttraining anhand dieser Kurve 15 bzw. anhand der von dieser ausgebildeten Extremstelle 16 kaum möglich sind. Der Stand der Technik kann nämlich lediglich einen Bereich von Massen bzw. Schätzungen vorgeben, mit denen ein bestimmtes Training erfolgen soll. Somit können, wie bereits ausgeführt, bei Verfahren des Stands der Technik individuelle Faktoren wie koordinative Fähigkeiten, das Zusammenspiel einzelner Muskeln einer Muskelgruppe etc. nicht oder nur eingeschränkt einfließen.

Die Erfindung zeigt jedoch anhand dieses Ausführungsbeispiels exakt jene Masse und jene Trainingsbedingungen, mit denen ein Kraft-Ausdauertraining erfolgen soll, da jene Teile des willkürlichen neuromuskulären Systems - Nerven, Muskelfasertypen etc. - in der optimalen Intensität wie gewünscht beansprucht werden. Es zeigt sich auch, dass in diesem Beispiel die Masse nach Extremstelle 2 um etwa 25% niedriger ist, als jene nach Extremstelle 16 - das Training hat also mit erheblich geringerer Masse zu erfolgen, als der Stand der Technik vermuten ließe. Auch wenn im Stand der Technik eine minimale zu erreichende Geschwindigkeit festgelegt wird, ist somit keine gezielte Trainingsfestlegung und/oder -steuerung möglich. Vielmehr kann es bei einem anhand einer Kurve 15 festgelegten Training also auch die Gefahr einer Fehl- oder Überbelastung bestehen.

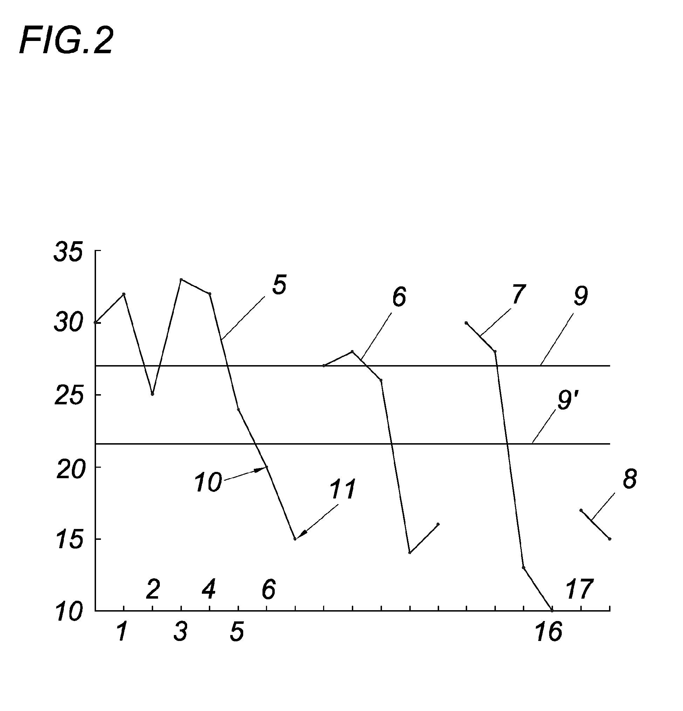

Des Weiteren wird erfindungsgemäß gezeigt, dass sich die Massen der ermittelten Extremstellen 2 und 4 bei diesem Probanden nur in vergleichsweise geringem Ausmaß voneinander unterscheiden. Das erfindungsgemäße Verfahren zeigt also entscheidende Verbesserungen gegenüber dem Stand der Technik, da durch dieses Leistungsfähigkeit, Trainingszustand etc. exakt abgestimmt werden können. Das Training wird erfindungsgemäß unter Vorgabe eines dabei zu erreichenden Ergebnisses der Funktion F bzw. der weitergebildeten Funktion F festgelegt. Es erfolgen durch den Probanden Serien an Wiederholungen einer Beschleunigung a einer Masse mit dem Ziel, jede Wiederholung mit maximaler Beschleunigung $a_{max}$ durchzuführen. Die tatsächlich erreichten Beschleunigungen a werden mittels Accelerometer bestimmt. Figur 2 zeigt eine graphische Darstellung eines Trainingsbeispiels und veranschaulicht die mögliche Festlegung und Steuerung eines Schnellkrafttrainings anhand der Überprüfung der Leistungsfähigkeit nach Kurve 3 in Figur 1. Entsprechend der Extremstelle 4 der Kurve 3 wird die zu beschleunigende Masse m mit 75 Kilogramm festgelegt. Das Trainingsbeispiel nach Fig. 2 beinhaltet Serien 5, 6, 7, 8 hintereinander ausgeführter Beschleunigungen einer Masse durch einen Probanden, wobei hierzu ausschließlich die über die Extremstelle 4 ermittelte Masse m verwendet wird. Die dabei erhaltenen Ergebnisse der Funktion F = m * $á_{max}$- werden als Punkt eingetragen und auf der X-Achse fortlaufend nummeriert. Als Ziel wird vorgegeben, diese Masse stets maximal zu beschleunigen, wobei der Zielwert dem Ergebnis der Extremstelle der Funktion F entsprechend vorgegeben wird. Insbesondere soll jedoch zumindest der Schwellwert 9', der 80% des Zielwerts entspricht, erreicht werden. Wie in Figur 2 ersichtlich, sinkt $á_{max}$ - die Serien des Trainings werden ja mit der gleichen Masse durchgeführt - mit Eintreten einer bestimmten Ermüdung des willkürlichen neuromuskulären Systems rasch und stark ab. Sobald zwei hintereinander folgende Beschleunigungen 10, 11 der Masse m unter diesen Schwellenwert 9' fallen, wird das Training daher unterbrochen. Nach einer Pause von drei Minuten wird eine weitere Serie mit denselben Vorgaben und demselben Schwellenwert 9' durchgeführt. Kann - wie in der Serie 8 - keine einzige Übungsausführung oberhalb des Schwellenwerts mehr durchgeführt werden, wird das Training abgebrochen, da dessen Fortsetzung wie bereits beschrieben nicht nur keinen zusätzlichen Trainingseffekt hinsichtlich Schnellkraft bringt, sondern diesbezüglich sogar negative Auswirkungen auf die körperliche Leistungsfähigkeit zu erwarten sind. Erfindungsgemäß werden also die Effektivität eines Trainings verbessert und vor allem werden auch nicht zielführende Belastungen und/oder Überbelastungen vermieden - womit das Training im Vergleich zu jenen des Stands der Technik vielfach bereits erheblich früher beendet wird. Bedeutung kommt diesen Vorteilen beispielsweise bei Probanden zu, die kein oder lediglich geringes Muskelwachstum wünschen. Gerade für Ausdauersportler ist ein günstiges Verhältnis von Kraft zu Körpergewicht von Vorteil. Somit ist es entscheidend, das Training gezielt auf die ausdauernde, schnellkräftige Muskelanteile abzustimmen und ein darüber hinausführendes evtl. Muskelhypertrophietraining zu vermeiden.

Der Vollständigkeit halber wird festgehalten, dass für ein erfindungsgemäßes Training selbstverständlich auch andere, von Masse m und ausschließlich ein von diesen erfassten Beschleunigungswerten abhängiger Maximalwert ($a_{max}$, $á_{max}$) herangezogen werden können. Ebenso ist vorstellbar, andere Kriterien etwa hinsichtlich des Schwellenwerts, erforderlicher Anzahl an Beschleunigungen, die unter diesem Schwellenwert liegen müssen, um das Training abzubrechen oder eventuell auch eine geringfügige Anpassung der Masse während des Trainings möglich sind. Dies hängt unter anderem von Leistungsfähigkeit des Probanden, Art und Zielsetzung des Trainings ab.

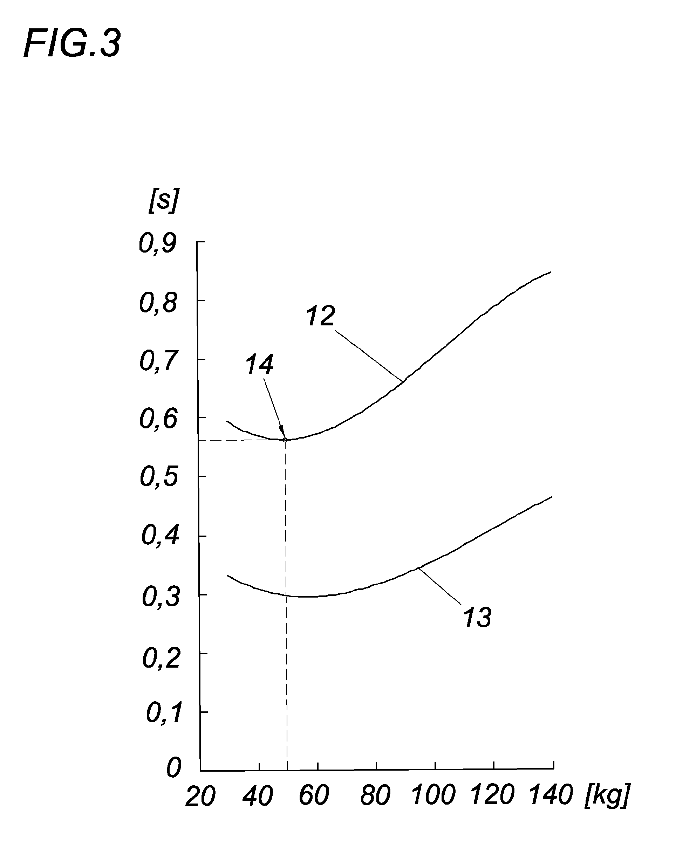

Figur 3 zeigt in einer grafischen Darstellung die interpolierten Ergebnisse der Zeitdauer auf der Y-Ache bis zur Erreichung der maximalen Beschleunigung $a_{max}$ einer bestimmten Masse auf der X-Achse als Kurve 12 und bis zur Erreichung der maximalen Ableitung der Beschleunigungen nach der Zeit, also des Maximalwerts $á_{max}$ der nach der Zeit (t) abgeleiteten Beschleunigungen (a) in Kurve 13 - jeweils entsprechend den Überprüfungen in der Figur 1. In Kurve 12 kann eine Extremstelle 14 erkannt werden, die anzeigt, dass die kürzeste Zeitdauer bis zur Erreichung der maximalen Beschleunigung $a_{max}$ bei 0,56 Sekunden liegt, welche bei Beschleunigung einer Masse von 49 Kilogramm erreicht wurde. Mithilfe dieser Überprüfung kann beispielsweise ein Training adaptiert oder - etwa anhand von Vergleichsdaten anderer Probanden - eventuelle Abweichungen von Normwerten erkannt werden. Sollte also gewünscht sein, ein Kraft-Ausdauer-Training insbesondere in Hinblick auf eine zusätzliche Verkürzung der Zeitdauer bis zur Erreichung des Ergebnisses aus der Funktion F zu adaptieren, besteht die Möglichkeit, dieses mit einer Masse m durchzuführen, die zwischen jener der in Fig. 1 ermittelten Extremstelle 2, also 79kg, und 49kg, also jener Masse m, mit der die kürzeste Zeitdauer bis zur Erreichung der maximalen Beschleunigung $a_{max}$ erreicht wurde. Als erster Schwellenwert werden höchstens 120% jener bei der gewählten Masse ermittelten Zeitdauer bis zur Erreichung der ihrer maximalen Beschleunigung $a_{max}$ festgelegt. Als zweiter Schwellenwert werden mindestens 80% des Ergebnisses dieser gewählten Masse festgelegt, die sich aus der Funktion F als Punkt der Kurve 1 errechnet. Für eine gezielte, maximale Verkürzung der Zeit bis zur Erreichung des Produkts in der Extremstelle 2 wird ein Training vorzugsweise im Bereich der Masse m entsprechend der Extremstelle 14 durchgeführt, was jedoch vom jeweiligen Trainingsziel abhängt.

Diese Vorgehensweise - unter anderem anhand der Kurve 13 - lässt sich selbstverständlich auch auf eine Adaptierung des Schnellkrafttrainings anwenden, vor allem, um die Zeitspanne bis zum Erreichen einer maximalen Dynamik, also $á_{max}$ zu verkürzen. Erfindungsgemäß können auf diese Weise also verschiedene Trainingsformen gezielt nach den jeweiligen Erfordernissen und Zielsetzungen adaptiert und optimiert, aber auch mögliche unerwünschte Entwicklungen in der Leistungsfähigkeit identifiziert werden.

Die Steigerung der Qualität des Trainings kann verbessert werden, indem zur Festlegung und/oder Steuerung des körperlichen Trainings eines Probanden Vergleichsdaten anderer Probanden herangezogen werden. So kann unter anderem festgestellt werden, unter welchen Bedingungen und unter welchen Zielsetzungen ein Proband trainiert hat, ob dessen Training fehlerhaft erfolgte etc. Des Weiteren kann auch eine Entwicklung der Leistungsfähigkeit vorausgesagt und eventuellen Fehlentwicklungen rechtzeitig entgegengewirkt werden. Eine Optimierung eines Verfahrens zur Festlegung und/oder Steuerung des körperlichen Trainings eines Probanden auf Grundlage wenigstens einer Untersuchung seiner körperlichen Leistungsfähigkeit ist somit zur Verfügung gestellt.

**Patentansprüche**

1. Verfahren zur Festlegung und/oder Steuerung des körperlichen Trainings eines Probanden auf Grundlage wenigstens einer Untersuchung seiner körperlichen Leistungsfähigkeit, bei welcher Untersuchung Beschleunigungen (a) bei unterschiedlichen Trainingswiderständen, insbesondere Beschleunigungen (a) unterschiedlicher Massen (m), erfasst werden und das Training in Abhängigkeit einer ermittelten Extremstelle (2) aus einer mathematischen Funktion (F), abhängig von diesen Trainingswiderständen, insbesondere der Größe der jeweiligen Masse (m), und den dazu erfassten Beschleunigungen (a), festgelegt und/oder gesteuert wird, **dadurch gekennzeichnet, dass** in der Funktion (F) von den erfassten Beschleunigungswerten ausschließlich ein von diesen abhängiger Maximalwert ($a_{max}$, $á_{max}$) herangezogen wird.

2. Verfahren nach Anspruch 1, **dadurch gekennzeichnet, dass** als Maximalwert ($a_{max}$) die maximale Beschleunigung verwendet wird.

3. Verfahren nach Anspruch 1, **dadurch gekennzeichnet, dass** als Maximalwert ($á_{max}$) der maximale Wert der nach der Zeit (t) abgeleiteten Beschleunigungen (a) verwendet wird.

4. Verfahren nach einem der Ansprüche 1 bis 3, **dadurch gekennzeichnet, dass** für die Bestimmung des Maximalwerts ($a_{max}$, $á_{max}$) Abweichungen der Bewegungsbahn am bzw. des Probanden von der Wirkrichtung des zu überwindenden Trainingswiderstands berücksichtigt werden.

5. Verfahren nach einem der Ansprüche 1 bis 4, **dadurch gekennzeichnet, dass** das Training unter Vorgabe eines zu erreichenden Maximalwerts ($a_{max}$, $á_{max}$) oder eines zu erreichenden Bereichs um diesen festgelegt wird.

6. Verfahren nach einem der Ansprüche 1 bis 5, **dadurch gekennzeichnet, dass** das Training unter Berücksichtigung einer Zeitdauer oder eines Zeitbereichs bis zur Erreichung eines Maximalwerts ($a_{max}$, $á_{max}$) festgelegt wird.

7. Verfahren nach einem der Ansprüche 1 bis 6, **dadurch gekennzeichnet, dass** zur Festlegung und/oder Steuerung des körperlichen Trainings eines Probanden Vergleichsdaten anderer Probanden herangezogen werden.

# FIG.1

FIG.2

FIG.3

Europäisches
Patentamt

European
Patent Office

Office européen
des brevets

**EUROPÄISCHER RECHERCHENBERICHT**

**Nummer der Anmeldung**

EP 13 18 4213

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2008/312560 A1 (JAMSEN ARI [FI] ET AL) 18. Dezember 2008 (2008-12-18) * Absätze [0012], [0027], [0050] - [0052], [0058] * * Abbildung 4 * ----- | 1-7 | INV. A61B5/22 ADD. A63B24/00 |
| X | US 2010/137107 A1 (JAEMSAE TIMO [FI] ET AL) 3. Juni 2010 (2010-06-03) * Absätze [0011], [0025], [0043], [0047] - [0049] * * Abbildung 4 * ----- | 1,2,5-7 | |
| A,D | EP 1 928 317 A1 (OULUN SEUDUN AMMATTIKORKEAKOUL [FI] OULUN SEUDUN AMMATTIKORKEAKOULU [F) 11. Juni 2008 (2008-06-11) * Abbildung 3 * ----- | 1-7 | |
| A | US 6 059 576 A (BRANN THEODORE L [US]) 9. Mai 2000 (2000-05-09) * Zusammenfassung * ----- | 4 | |

**RECHERCHIERTE SACHGEBIETE (IPC)**

A61B
A63B

Der vorliegende Recherchenbericht wurde für alle Patentansprüche erstellt

| Recherchenort | Abschlußdatum der Recherche | Prüfer |
|---|---|---|
| Den Haag | 8. Januar 2014 | Worms, Georg |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

...................................................................................................
& : Mitglied der gleichen Patentfamilie, übereinstimmendes Dokument

EPO FORM 1503 03.82 (P04C03)

**EP 2 708 186 A1**

## ANHANG ZUM EUROPÄISCHEN RECHERCHENBERICHT
## ÜBER DIE EUROPÄISCHE PATENTANMELDUNG NR.

EP 13 18 4213

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-01-2014

| Im Recherchenbericht angeführtes Patentdokument | Datum der Veröffentlichung | Mitglied(er) der Patentfamilie | Datum der Veröffentlichung |
|---|---|---|---|
| US 2008312560 A1 | 18-12-2008 | EP 1768559 A1 | 04-04-2007 |
| | | FI 20040773 A | 05-12-2005 |
| | | JP 2008501389 A | 24-01-2008 |
| | | US 2008312560 A1 | 18-12-2008 |
| | | WO 2005117703 A1 | 15-12-2005 |
| US 2010137107 A1 | 03-06-2010 | US 2010137107 A1 | 03-06-2010 |
| | | WO 2008071840 A1 | 19-06-2008 |
| EP 1928317 A1 | 11-06-2008 | AT 499879 T | 15-03-2011 |
| | | EP 1928317 A1 | 11-06-2008 |
| | | WO 2007036611 A1 | 05-04-2007 |
| US 6059576 A | 09-05-2000 | AU 1421299 A | 15-06-1999 |
| | | CA 2311559 A1 | 03-06-1999 |
| | | EP 1032459 A1 | 06-09-2000 |
| | | JP 2001523536 A | 27-11-2001 |
| | | US 6059576 A | 09-05-2000 |
| | | WO 9926704 A1 | 03-06-1999 |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20110207581 A1
- US 20090069722 A1
- US 20120094804 A1
- WO 2007036611 A1